# EUROPEAN PATENT APPLICATION

(11) **EP 4 454 694 A1**
(43) Date of publication of application: **30.10.2024**
(21) Application number: 22911037.4
(22) Date of filing: 14.12.2022
(51) Int. Cl.: A61M 25/00, A61M 25/092, A61M 25/098

(54) **MEDICAL DEVICE**

(30) Priority: 24.12.2021 JP 2021211223
(71) Applicant: SB-Kawasumi Laboratories, Inc., Kawasaki-shi, Kanagawa 210-8602 (JP); Sumitomo Bakelite Co.Ltd., Shinagawa-ku Tokyo 140-0002 (JP)
(72) Inventor: FUJITA Yasuhiro, Kawasaki-shi, Kanagawa 210-8602 (JP); YAMAGUCHI Kenjiro, Kawasaki-shi, Kanagawa 210-8602 (JP); HAYASHI Nobuaki, Kawasaki-shi, Kanagawa 210-8602 (JP)
(74) Representative: Müller-Boré & Partner Patentanwälte PartG mbB
(86) International application number: PCT/JP2022/046061
(87) International publication number: WO 2023/120329

(57) **Abstract**

The present invention provides a medical device having a structure with excellent operability. According to the present invention, there is provided a medical device (100) including: an elongated medical device body (10); a first operation wire (41) and a second operation wire (42), each of which extends along a longitudinal direction of the medical device body (10); and an operation part (70) that is configured to individually and simultaneously pull the first operation wire (41) and the second operation wire (42), in which the first operation wire (41) and the second operation wire (42) are disposed at opposite extreme positions in a transverse section of the medical device body (10). A distal end of the first operation wire (41) is fixed to a distal end part (10a) of the medical device body (10), and a distal end of the second operation wire (42) is fixed to the distal end part (10a) of the medical device body (10) at a position on a proximal end side with respect to the distal end of the first operation wire (41).

## Description

### Technical Field

The present invention relates to a medical device. Priority is claimed on Japanese Patent Application No. 2021-211223, filed on December 24, 2021, the content of which is incorporated herein by reference.

### Background Art

As a medical device capable of a bending operation of a distal end part, a type of medical device including an operation wire is known (for example, PTL 1).

In a medical device of PTL 1, a plurality of hollow tubes are disposed around a central lumen, and operation wires are respectively inserted into two hollow tubes facing each other with the central lumen interposed therebetween. In the medical device of PTL 1, a distal end of the operation wire is fixed to a distal end part of the catheter. The medical device of PTL 1 is configured to perform a pulling operation of a rear end of the operation wire. Consequently, the distal end part of the catheter can be bent by selecting and pulling the operation wire.

### Citation List

### Patent Literature

[PTL 1] Japanese Unexamined Patent Application, First Publication No. 2013-48711

### Summary of Invention

### Technical Problem

However, in the medical device of PTL 1, there is still room for improvement in terms of operability of the medical device.

The present invention has been made in view of the above-described problem and provides a medical device having a structure with more excellent operability.

### Solution to Problem

According to the present invention, there is provided a medical device including:
an elongated medical device body;
a first operation wire and a second operation wire, each of which extends along a longitudinal direction of the medical device body; and
an operation part that is configured to individually and simultaneously pull the first operation wire and the second operation wire,
in which the first operation wire and the second operation wire are disposed at opposite extreme positions in a transverse section of the medical device body,
a distal end of the first operation wire is fixed to a distal end part of the medical device body, and
a distal end of the second operation wire is fixed to the distal end part of the medical device body at a position on a proximal end side with respect to the distal end of the first operation wire.

### Advantageous Effects of Invention

According to the present invention, it is possible to further improve operability of the medical device.

### Brief Description of Drawings

FIG. 1A is a view showing a distal end part of a medical device according to a first embodiment and is a view showing a state in which a bending operation is not performed.
FIG. 1B is a view showing the distal end part of the medical device according to the first embodiment and is a view showing a state in which the bending operation is performed.
FIG. 2 is a longitudinal sectional view of a medical device body in the first embodiment.
FIG. 3 is a transverse sectional view of the medical device body taken along line A-A of FIG. 2.
FIG. 4 is a transverse sectional view of the medical device body taken along line B-B of FIG. 2.
FIG. 5 is a side view showing an operation part of the medical device according to the first embodiment and a portion near the operation part.
FIG. 6 is a plan view showing the operation part of the medical device according to the first embodiment and a portion near the operation part.
FIG. 7A is a side view of a tension release mechanism in the first embodiment.
FIG. 7B is a side view of a first rotation member in the first embodiment.
FIG. 7C is a view showing a state in which an engagement recessed portion and an engagement protruding portion in the first embodiment are engaged with each other.
FIG. 7D is a view showing a state in which the engagement between the engagement protruding portion and the engagement recessed portion in the first embodiment is released.
FIG. 8 is a schematic view in which each of (a), (b), and (c) illustrates a bending motion of the distal end part of the medical device body in the first embodiment.
FIG. 9 is a schematic view in which (a) and (b) illustrate bending motions of the distal end part of the medical device body in the first embodiment.
FIG. 10 is a view showing a distal end part of a medical device according to a second embodiment.
FIG. 11 is a longitudinal sectional view of a medical device body in the second embodiment.
FIG. 12 is a transverse sectional view of the medical device body taken along line A-A of FIG. 11.
FIG. 13 is a plan view showing an operation part of the medical device according to the second embodiment and a portion near the operation part.
FIG. 14 is a longitudinal sectional view of a medical device body in a third embodiment.
FIG. 15 is a sectional view taken along line A-A of FIG. 14.
FIG. 16 is a plan view showing an operation part of the medical device according to the third embodiment and a portion near the operation part.
FIG. 17A is a schematic view by a plan view illustrating a bending motion of a distal end part of the medical device body in the third embodiment.
FIG. 17B is a view illustrating a bending motion of the distal end part of the medical device body in the third embodiment and is a view as viewed in a direction of an arrow A shown in FIG. 17A.
FIG. 17C is a view illustrating a bending motion of the distal end part of the medical device body in the third embodiment and is a view as viewed in a direction of an arrow B shown in FIG. 17A.
FIG. 18 is a schematic view illustrating a bending motion of the distal end part of the medical device body in the third embodiment.

### Description of Embodiments

Hereinafter, embodiments of the present invention will be described using the drawings. In all the drawings, the same components are denoted by the same reference numerals, and descriptions thereof will be omitted as appropriate.

Various components of a catheter according to the present embodiment are not necessarily independent entities, and a plurality of components formed as one member, one component formed of a plurality of members, a certain component as a part of another component, overlapping parts between a certain component and another component, and the like are allowed.

The terms used in the description of the present embodiment are defined as follows unless otherwise specified.

A distal end part is an end part (distal end) on an insertion distal end side of a medical device 100 in each part of the medical device 100, and a proximal end part is an end part (proximal end part) on a proximal end side of the medical device 100 in each part of the medical device 100.

In addition, an axial core means a central axis along a longitudinal direction of a medical device body 10.

A longitudinal section of the medical device body 10 refers to a section obtained by cutting the medical device body 10 along the axial core.

A transverse section of the medical device body 10 refers to a section obtained by cutting the medical device 100 along a plane orthogonal to the axial core.

### [First Embodiment]

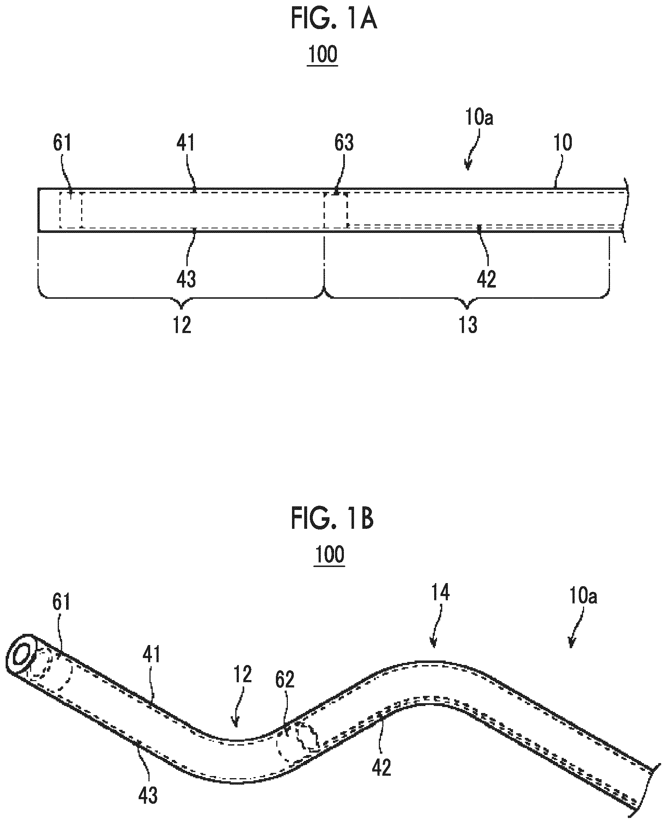

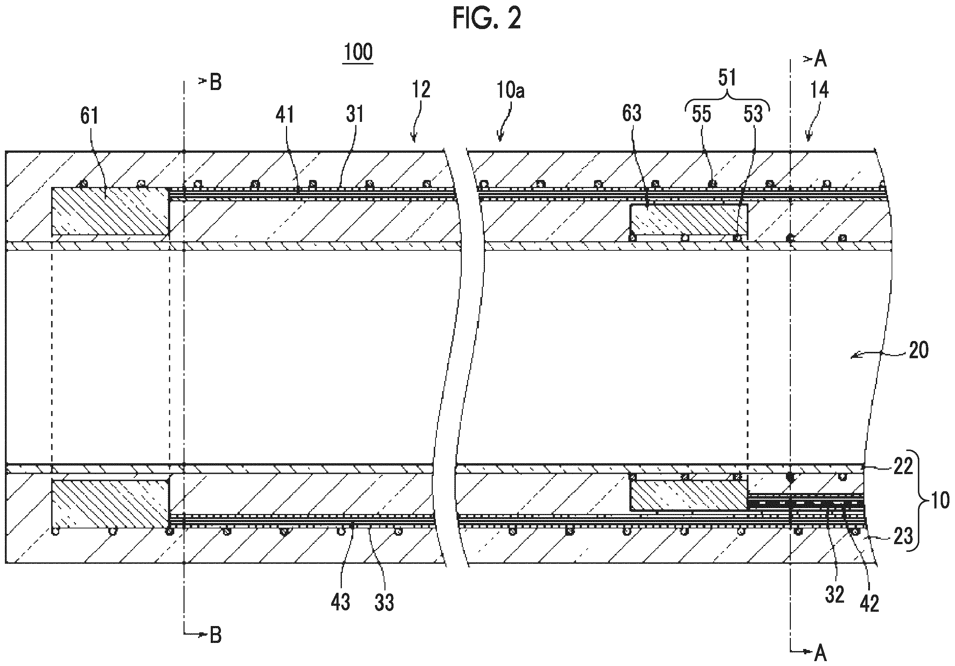

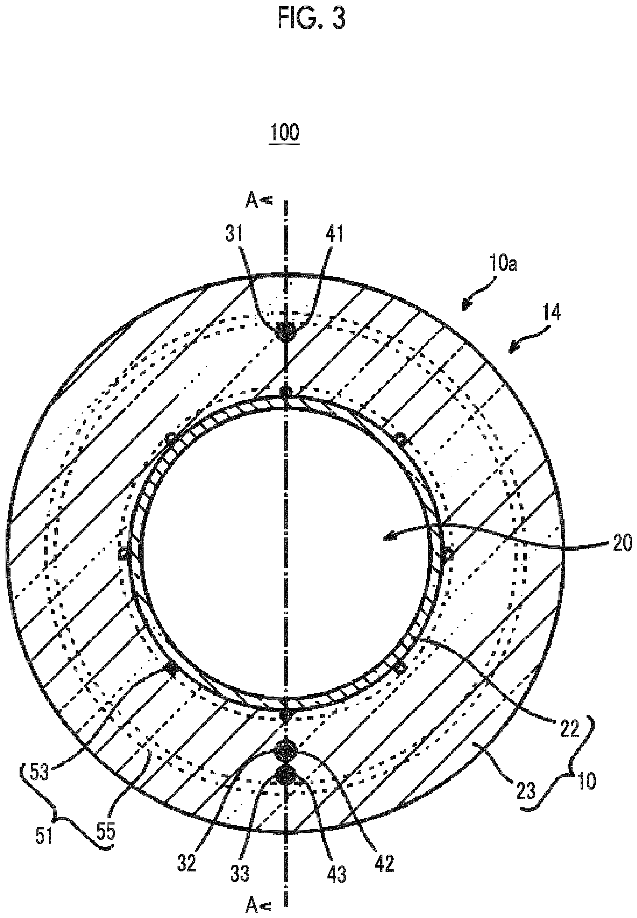

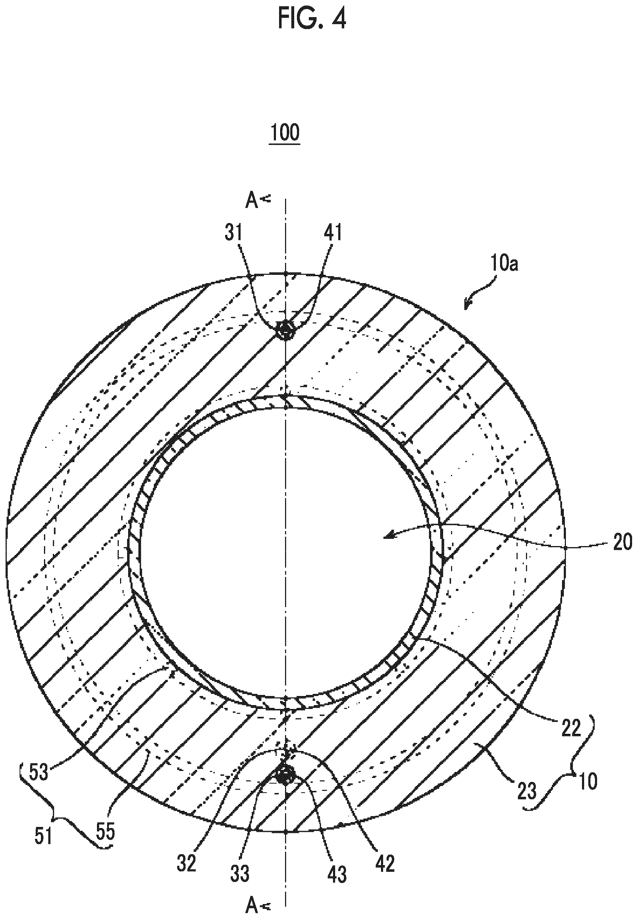

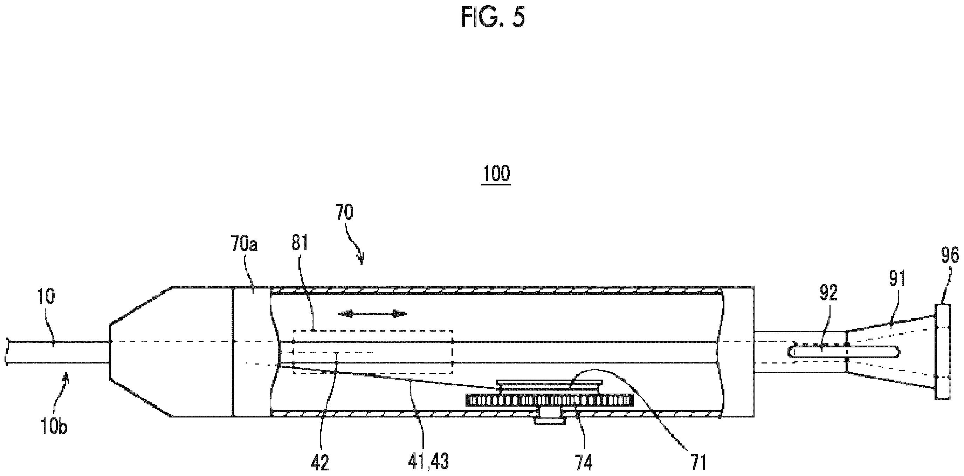

First, a first embodiment will be described using FIGS. 1A to 8. In FIGS. 1A and 1B, a first operation wire 41 to a third operation wire 43 are shown by dashed lines. In addition, FIG. 2 is a sectional view taken along line A-A of FIG. 3. In FIG. 5, a body case 70a of an operation part 70 is partially broken to show a structure inside the body case 70a.

As shown in FIGS. 1A, 2, and 3, the medical device 100 according to the present embodiment includes the elongated medical device body 10, the first operation wire 41 and the second operation wire 42, each of which extends along the longitudinal direction of the medical device body 10, and the operation part 70 that is configured to individually and simultaneously pull the first operation wire 41 and the second operation wire 42.

The first operation wire 41 and the second operation wire 42 are disposed at opposite extreme positions in the transverse section of the medical device body 10.

A distal end of the first operation wire 41 is fixed to a distal end part 10a of the medical device body 10, and a distal end of the second operation wire 42 is fixed to the distal end part 10a of the medical device body 10 at a position on the proximal end side with respect to the distal end of the first operation wire 41.

Here, the opposite extreme positions mean that the first operation wire 41 and the second operation wire 42 are spaced apart from each other by 160 degrees or more in a circumferential direction of the medical device body 10, and preferably, the first operation wire 41 and the second operation wire 42 are spaced apart from each other by 170 degrees or more in the circumferential direction of the medical device body 10.

According to the present embodiment, by selectively pulling the first operation wire 41 out of the first operation wire 41 and the second operation wire 42, the distal end part 10a of the medical device body 10 can be selectively bent in one direction (hereinafter, a first direction). Further, by simultaneously pulling the first operation wire 41 and the second operation wire 42, it is possible to primarily bend a region (hereinafter, a second region 14) on the proximal end side with respect to the distal end of the second operation wire 42 in a second direction while primarily bending a region (hereinafter, a first region 12) on the distal end side with respect to the distal end of the second operation wire 42 in the first direction, in the distal end part 10a of the medical device body 10, as shown in FIG. 1B. In addition, by selectively pulling the second operation wire 42 out of the first operation wire 41 and the second operation wire 42, the distal end part 10a of the medical device body 10 can be bent in a direction (second direction) opposite to the first direction. That is, since the medical device body 10 can be bent with a higher degree of freedom according to a branching direction of a branching part of a body cavity such as a blood vessel, the branching selectivity of the medical device 100 can be improved. Therefore, the operability of the medical device 100 can be improved.

In the case of the present embodiment, the medical device 100 is, as an example, a catheter. More specifically, for example, the medical device 100 is an intravascular catheter used by inserting the medical device body 10 into a blood vessel. However, the medical device 100 may be, for example, a guide wire used when inserting the catheter into a body cavity.

In the case of the present embodiment, as shown in FIGS. 1A, 2, and 3, the medical device 100 further includes a third operation wire 43 that extends along the longitudinal direction of the medical device body 10.

The third operation wire 43 is disposed at a position near the second operation wire 42 in the transverse section of the medical device body 10, and a distal end of the third operation wire 43 is fixed to the distal end part 10a of the medical device body 10 at a position on the distal end side with respect to the distal end of the second operation wire 42.

With such a configuration, by pulling the third operation wire 43, the distal end part 10a of the medical device body 10 can be bent in a direction substantially opposite to a direction in which the distal end part 10a of the medical device body 10 is bent by pulling the first operation wire 41.

Here, being disposed at a position near the second operation wire 42 means being positioned within 20 degrees from a position of the second operation wire 42 in the circumferential direction of the medical device body 10, preferably being positioned within 10 degrees from the position of the second operation wire 42.

In the circumferential direction of the medical device body 10, the first operation wire 41, and the second operation wire 42 and the third operation wire 43 are disposed at opposite extreme positions.

In addition, since the third operation wire 43 is disposed at a position near the second operation wire 42 in the transverse section of the medical device body 10, the first operation wire 41 and the third operation wire 43 are disposed at opposite extreme positions in the transverse section of the medical device body 10.

Here, the opposite extreme positions mean that the first operation wire 41 and the third operation wire 43 are spaced apart from each other by 160 degrees or more in the circumferential direction of the medical device body 10, and preferably, the first operation wire 41 and the third operation wire 43 are spaced apart from each other by 170 degrees or more in the circumferential direction of the medical device body 10.

More specifically, in the case of the present embodiment, for example, as shown in FIGS. 2 and 3, the first operation wire 41 and the second operation wire 42 are disposed at positions that are 180 degrees rotationally symmetric to each other with respect to an axial center of the medical device body 10 as a reference. In addition, the second operation wire 42 and the third operation wire 43 are disposed at substantially equivalent positions in the circumferential direction of the medical device body 10 with the axial center of the medical device body 10 as a reference. Therefore, the third operation wire 43 and the first operation wire 41 are disposed at positions that are 180 degrees rotationally symmetric to each other with respect to the axial center of the medical device body 10 as a reference.

In the case of the present embodiment, a direction in which the distal end part 10a of the medical device body 10 is bent by pulling the first operation wire 41 and a direction in which the distal end part 10a of the medical device body 10 is bent by pulling the third operation wire 43 or the second operation wire 42 are directions opposite to each other.

The first operation wire 41, the second operation wire 42, and the third operation wire 43 are each made of a thin wire such as a metal or a resin.

Here, in the case of the present embodiment, a breaking strength of the second operation wire 42 is greater than a breaking strength of the first operation wire 41.

Consequently, the second operation wire 42 can be more strongly pulled.

More specifically, in the case of the present embodiment, the first operation wire 41 and the second operation wire 42 are made of, for example, the same type of material. Meanwhile, by setting a wire diameter of the second operation wire 42 to a dimension larger than a wire diameter of the first operation wire 41, the breaking strength of the second operation wire 42 can be made greater than the breaking strength of the first operation wire 41. However, for example, by using a material having a greater breaking strength than a breaking strength of a material constituting the first operation wire 41 as a material constituting the second operation wire 42, the breaking strength of the second operation wire 42 can also be made greater than the breaking strength of the first operation wire 41.

In addition, in the case of the present embodiment, as an example, the third operation wire 43 and the first operation wire 41 are made of the same type of material, and a wire diameter of the third operation wire 43 is set to, for example, a substantially equivalent dimension to the wire diameter of the first operation wire 41.

For example, the wire diameter of the third operation wire 43 and the wire diameter of the first operation wire 41 may be set to different dimensions from each other. Further, the third operation wire 43 and the first operation wire 41 may be made of, for example, different types of materials from each other.

The wire diameter of the first operation wire 41 and the wire diameter of the third operation wire 43 are not particularly limited, but are preferably 10 µm or more and 60 µm or less and more preferably 20 µm or more and 50 µm or less.

The wire diameter of the second operation wire 42 is not particularly limited, but is preferably 30 µm or more and 180 µm or less, and more preferably 60 µm or more and 150 µm or less.

Note that, as described above, it is preferable that the wire diameter of the second operation wire 42 is larger than the wire diameter of the first operation wire 41.

In the case of the present embodiment, the medical device body 10 has a lumen 20 that is open at a distal end of the medical device body 10.

As shown in FIGS. 2 and 3, the medical device body 10 has, for example, a two-layer structure including an inner layer 22 and an outer layer 23 provided around the inner layer 22 and is configured by being laminated in the order of the inner layer 22 and the outer layer 23 from an axial core side of the medical device body 10.

The inner layer 22 is an innermost layer of the medical device body 10 and is formed, for example, in a circular tubular shape having a constant wall thickness regardless of the position in an axial direction. The inner layer 22 is open at both ends, that is, the distal end and the proximal end of the medical device body 10. In the case of the present embodiment, the lumen 20 is defined by an inner peripheral surface of the inner layer 22.

The inner layer 22 is made of, for example, a fluorine-based or olefin-based thermoplastic polymer resin. The fluorine-based thermoplastic polymer material is not particularly limited and can include polytetrafluoroethylene (PTFE), polyvinylidene fluoride (PVDF), and perfluoroalkoxy fluororesin (PFA), and similarly, the olefin-based polymer material can include polyethylene and the like.

The outer layer 23 is an outermost layer of the medical device body 10. For example, the majority (more than half) of a wall thickness of the medical device body 10 is occupied by a wall thickness of the outer layer 23. The outer layer 23 is formed, for example, in a circular tubular shape having a substantially constant wall thickness regardless of the position in the axial direction. However, an outer diameter of a distal end of a distal end part of the outer layer 23 is slightly decreased toward the distal end side, for example.

A hydrophilic coating may be formed on an outer surface layer of the medical device body 10 as necessary.

The lumen 20 is continuously formed from the distal end to the proximal end of the medical device body 10 and is open at the distal end and the proximal end of the medical device body 10, respectively.

As shown in FIGS. 2 to 4, the medical device body 10 further includes a first hollow tube 31, a second hollow tube 32, and a third hollow tube 33 that are each embedded in the outer layer 23.

The first operation wire 41 is inserted through the first hollow tube 31, the second operation wire 42 is inserted through the second hollow tube 32, and the third operation wire 43 is inserted through the third hollow tube 33.

The first hollow tube 31, the second hollow tube 32, and the third hollow tube 33 are each a sub-lumen tube, and the inner lumens of these sub-lumen tubes are sub-lumens. That is, each of the operation wires (the first operation wire 41, the second operation wire 42, and the third operation wire 43) is inserted through the sub-lumen.

Inner diameters of the first hollow tube 31, the second hollow tube 32, and the third hollow tube 33 are smaller than the inner diameter of the lumen 20.

In the case of the present embodiment, the second operation wire 42 and the second hollow tube 32, and the third operation wire 43 and the third hollow tube 33 are disposed side by side in a radial direction of the medical device body 10. The third operation wire 43 and the third hollow tube 33 are disposed radially outward of the second operation wire 42 and the second hollow tube 32 (at a position away from the axial core of the medical device body 10) in the medical device body 10.

However, for example, the second operation wire 42 and the second hollow tube 32, and the third operation wire 43 and the third hollow tube 33 may be disposed side by side in the circumferential direction of the medical device body 10. In addition, in the radial direction, a spaced distance between the axial core of the medical device body 10 and an axial core of the second operation wire 42 (or an axial core of the second hollow tube 32) may be, for example, substantially equivalent to a spaced distance between the axial core of the medical device body 10 and an axial core of the third operation wire 43 (or an axial core of the third hollow tube 33).

As materials of the first hollow tube 31, the second hollow tube 32, and the third hollow tube 33, for example, a resin material such as polytetrafluoroethylene (PTFE), polyvinylidene fluoride (PVDF), or perfluoroalkoxy fluororesin (PFA), polyethylene, or the like can be used.

As shown in FIGS. 1A and 2, a ring-shaped first marker 61 made of a radiopaque metal material is embedded in the distal end part 10a of the medical device body 10.

The first marker 61 is disposed coaxially with the lumen 20 and around the lumen 20.

The first marker 61 is disposed, for example, around a reinforcing layer 51.

Further, in the medical device body 10, a ring-shaped second marker 63 made of a radiopaque metal material is embedded in a part on the proximal end side with respect to a part where the first marker 61 is embedded.

The second marker 63 is disposed coaxially with the lumen 20 and around the lumen 20.

The second marker 63 is disposed, for example, around the reinforcing layer 51.

For example, a through-hole (not shown) that penetrates a wall thickness portion of the second marker 63 in the axial direction may be formed in the second marker 63. In addition, for example, a portion of the second marker 63 in the circumferential direction may be interrupted, and the second marker 63 may have a substantially C-like shape when viewed in the axial direction.

For example, the first marker 61 and the second marker 63 have the same inner diameter. For example, an outer diameter of the first marker 61 is larger than an outer diameter of the second marker 63.

The distal end of the first operation wire 41 and the distal end of the first hollow tube 31 are fixed to a proximal end of the first marker 61 by, for example, soldering, welding, a mechanical coupling that generates frictional resistance, or the like (not shown).

The distal end of the second operation wire 42 and the distal end of the second hollow tube 32 are fixed to a proximal end of the second marker 63 by, for example, soldering, welding, a mechanical coupling that generates frictional resistance, or the like (not shown).

The distal end of the third operation wire 43 and the distal end of the third hollow tube 33 are fixed to the proximal end of the first marker 61 by, for example, soldering, welding, a mechanical coupling that generates frictional resistance, or the like (not shown).

Therefore, the distal end of the first operation wire 41 and the distal end of the third operation wire 43 are disposed at the same position (substantially the same position) in the axial direction of the medical device body 10.

As described above, the medical device 100 includes the first marker 61 that is fixed to the distal end part 10a of the medical device body 10 and the second marker 63 that is fixed to the distal end part 10a of the medical device body 10 at a position on the proximal end side with respect to the first marker 61, and the distal end of the first operation wire 41 and the distal end of the third operation wire 43 are fixed to the first marker 61, and the distal end of the second operation wire 42 is fixed to the second marker 63.

In the present invention, the number of markers provided in the medical device 100 may be, for example, two or more.

In the case of the present embodiment, in the distal end part 10a of the medical device body 10, a region on the distal end side with respect to a distal end of the second marker 63 is the first region 12, and a region on the proximal end side with respect to the distal end of the second marker 63 is the second region 14. In the distal end part 10a, a length of the first region 12 and a length of the second region 14 can be adjusted by setting the distance between the first marker 61 and the second marker 63 in the axial direction of the medical device body 10 to a desired distance.

In addition, the distal end part of the first operation wire 41 is indirectly fixed to the distal end part 10a of the medical device body 10 by being fixed to the first marker 61, and the distal end part of the third operation wire 43 is indirectly fixed to the distal end part 10a of the medical device body 10 by being fixed to the second marker 63.

As shown in FIGS. 1A and 2, the first operation wire 41 is routed from a region on the proximal end side with respect to the second marker 63 to a region on the distal end side with respect to the second marker 63 through a portion of the medical device body 10 on a radially outer side with respect to a disposition region of the second marker 63.

More specifically, the first hollow tube 31 through which the first operation wire 41 is inserted is also routed from the region on the proximal end side with respect to the second marker 63 to the region on the distal end side with respect to the second marker 63 through the portion of the medical device body 10 on the radially outer side with respect to the disposition region of the second marker 63.

However, in the present invention, a spaced distance in the radial direction between the axial core of the medical device body 10 and the axial core of the first operation wire 41 (or the axial core of the first hollow tube 31) may be, for example, substantially equivalent to the spaced distance between the axial core of the medical device body 10 and the axial core of the second operation wire 42 (or the axial core of the second hollow tube 32).

Similarly, the third operation wire 43 is routed from the region on the proximal end side with respect to the second marker 63 to the region on the distal end side with respect to the second marker 63 through the portion of the medical device body 10 on the radially outer side with respect to the disposition region of the second marker 63.

More specifically, the third hollow tube 33 through which the third operation wire 43 is inserted is also routed from the region on the proximal end side with respect to the second marker 63 to the region on the distal end side with respect to the second marker 63 through the portion of the medical device body 10 on the radially outer side with respect to the disposition region of the second marker 63.

However, in the present invention, the spaced distance in the radial direction between the axial core of the medical device body 10 and the axial core of the third operation wire 43 (or the axial core of the third hollow tube 33) may be, for example, substantially equivalent to the spaced distance between the axial core of the medical device body 10 and the axial core of the second operation wire 42 (or the axial core of the second hollow tube 32).

Here, in the case of the present embodiment, the medical device body 10 includes the reinforcing layer 51. A bending stiffness of the reinforcing layer 51 on the proximal end side with respect to the second marker 63 is greater than a bending stiffness of the reinforcing layer 51 on the distal end side with respect to the second marker 63. A bending stiffness of the medical device body 10 on the proximal end side with respect to the second marker 63 is greater than a bending stiffness of the medical device body 10 on the distal end side with respect to the second marker 63.

More specifically, as shown in FIGS. 2 and 3, the reinforcing layer 51 includes, for example, a blade layer 53 embedded in the outer layer 23 and a winding wire 55 embedded in the outer layer 23. Each of the blade layer 53 and the winding wire 55 extends from the distal end side to a proximal end part 10b of the medical device body 10. However, as shown in FIG. 2, a distal end of the blade layer 53 is disposed, for example, at a position substantially equivalent to a position of the distal end of the second marker 63 in the axial direction. Meanwhile, a distal end of the winding wire 55 is disposed, for example, at a position substantially equivalent to a position of a distal end of the first marker 61 in the axial direction.

Consequently, in the medical device body 10, the region on the proximal end side with respect to the second marker 63 is reinforced by the blade layer 53 and the winding wire 55, and the region on the distal end side with respect to the second marker 63 is reinforced by the winding wire 55. Therefore, the bending stiffness of the medical device body 10 on the proximal end side with respect to the second marker 63 is greater than the bending stiffness of the medical device body 10 on the distal end side with respect to the second marker 63.

The blade layer 53 is formed by braiding a plurality of wires. The blade layer 53 is disposed, for example, around the inner layer 22.

The first hollow tube 31, the second hollow tube 32, and the third hollow tube 33 are disposed, for example, radially outward of the reinforcing layer 51 (at a position away from the axial core of the medical device body 10) in the medical device body 10.

A material of the wire constituting the blade layer 53 is preferably, for example, a metal material such as stainless steel or tungsten, but may be a resin material.

The winding wire 55 is wound around the medical device body 10 radially outward of the reinforcing layer 51, the first hollow tube 31, the second hollow tube 32, and the third hollow tube 33.

A material of the wire constituting the winding wire 55 is preferably, for example, a metal material such as stainless steel or tungsten, but may be a resin material.

In the present invention, the distal end of the blade layer 53 may be disposed, for example, at a position substantially equivalent to the position of the distal end of the first marker 61 (the position of the distal end of the winding wire 55). In this case, for example, it is preferable that the blade layer 53 in the first region 12 is composed of a fewer number of wires than the number of wires in the second region 14, or that an opening width of the mesh of the blade layer 53 in the first region 12 is larger than an opening width of the mesh of the blade layer 53 in the second region 14. By doing so, a configuration can be employed in which the bending stiffness of the medical device body 10 on the proximal end side with respect to the second marker 63 is greater than the bending stiffness of the medical device body 10 on the distal end side with respect to the second marker 63.

In addition, in the present invention, the reinforcing layer 51 may include, for example, a coil layer (not shown) formed by winding a wire a plurality of times, instead of the blade layer 53.

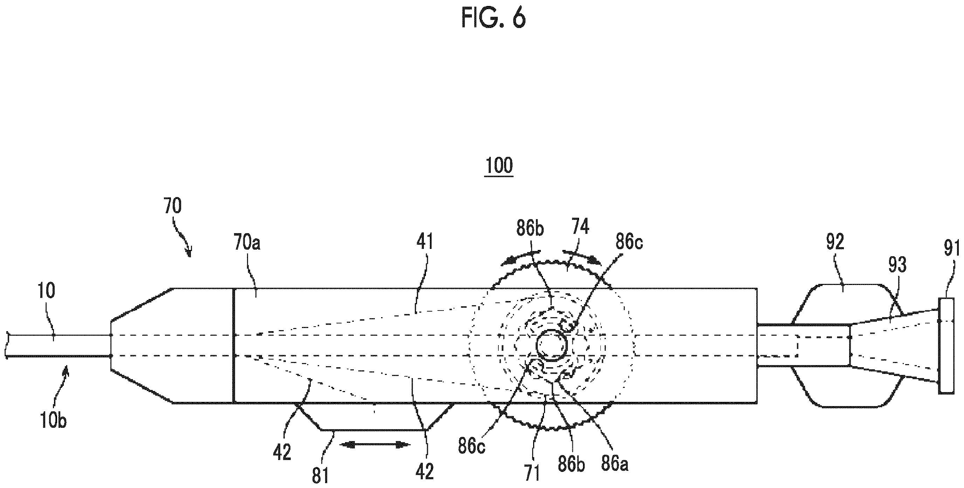

As shown in FIGS. 5 and 6, in the case of the present embodiment, the medical device 100 includes a hub 91 provided at the proximal end part of the medical device body 10.

The hub 91 includes a coupling portion 93 for inserting an injector (syringe) (not shown) into a proximal end of the hub 91. A screw groove is formed on an outer periphery of the coupling portion 93 such that the syringe can be attachably and detachably fixed.

The hub 91 includes two blade portions 92 that face each other with the axial core of the hub 91 interposed therebetween and that are provided on an outer periphery of the hub 91.

The proximal end part of the medical device body 10 is inserted into and fixed to a distal end part of the hub 91. Consequently, the lumen 20 inside the medical device body 10 and an internal space of the hub 91 communicate with each other.

By rotating the blade portions 92 about the axial core of the hub 91, it is possible to perform a torque operation of rotating the entire medical device body 10 about the axis.

The body case 70a of the operation part 70, which will be described below, is connected and fixed to the distal end side of the hub 91.

As described above, the medical device 100 includes the operation part 70 for performing the bending operation of the distal end part 10a of the medical device body 10. With respect to the operation part 70, an operation of selectively pulling one of the first operation wire 41 or the third operation wire 43, and an operation of individually and simultaneously pulling the first operation wire 41 and the second operation wire 42 are possible.

As shown in FIGS. 5 and 6, the operation part 70 includes, for example, the body case 70a and a first rotation member 71 that is pivotally supported to be rotatable with respect to the body case 70a.

The first rotation member 71 is, for example, a pulley.

Here, in the present specification, a certain member being rotatable is not limited to an aspect in which the member is rotatable by 360 degrees or more, but also includes an aspect in which the member is only swingable in a predetermined angular range of less than 360 degrees.

More specifically, a first dial operation portion 74 having a disc-like shape or the like is engaged with the first rotation member 71 coaxially with the first rotation member 71. Since the first dial operation portion 74 is pivotally supported to be rotatable with respect to the body case 70a, the first rotation member 71 is indirectly and pivotally supported to be rotatable with respect to the body case 70a.

The proximal end part 10b of the medical device body 10 is led to the proximal end side of the body case 70a through the inside of the body case 70a, and is inserted into and fixed to the distal end part of the hub 91.

A rotary shaft of the first dial operation portion 74 extends in a direction orthogonal to an axial core direction of the medical device body 10 in the body case 70a.

As shown in FIG. 6, at least a portion of the first dial operation portion 74 is exposed to the outside of the body case 70a, and an operator who operates the medical device 100 can operate the first dial operation portion 74 from the outside of the body case 70a.

Further, the operation part 70 includes, for example, a first slider operation portion 81 that is fixed to the body case 70a to be slidable in the axial direction.

A slide hole (not shown) that extends in the axial direction is formed in the body case 70a, and the first slider operation portion 81 is guided by the slide hole and is slidable in the axial direction relative to the body case 70a.

As shown in FIG. 6, at least a portion of the first slider operation portion 81 is exposed to the outside of the body case 70a via the slide hole, and the operator who operates the medical device 100 can perform an operation of sliding the first slider operation portion 81 toward the proximal end side or the distal end side from the outside of the body case 70a.

As shown in FIG. 5, each of the first operation wire 41, the second operation wire 42, and the third operation wire 43 is led out from the medical device body 10 in the body case 70a.

A proximal end part of the first operation wire 41 is, for example, wound around the first rotation member 71 by one and a half turns, and a proximal end of the first operation wire 41 is fixed to the first rotation member 71.

In addition, a proximal end part of the third operation wire 43 is, for example, wound around the first rotation member 71 by one and a half turns, and a proximal end of the third operation wire 43 is fixed to the first rotation member 71.

A winding direction of the first operation wire 41 and a winding direction of the third operation wire 43 with respect to the first rotation member 71 are directions opposite to each other.

In a case where the first rotation member 71 that is engaged with the first dial operation portion 74 is rotated in one direction by a rotation operation with respect to the first dial operation portion 74, the first operation wire 41 out of the first operation wire 41 and the third operation wire 43 is selectively pulled toward the proximal end side.

In addition, in a case where the first rotation member 71 is rotated in a direction opposite to the above-described one direction by the rotation operation with respect to the first dial operation portion 74, the third operation wire 43 out of the first operation wire 41 and the third operation wire 43 is selectively pulled toward the proximal end side.

A proximal end of the second operation wire 42 is fixed to, for example, the first slider operation portion 81.

In a case where the first slider operation portion 81 is moved toward the proximal end side relative to the body case 70a in the axial direction through a sliding operation with respect to the first slider operation portion 81, the second operation wire 42 is pulled toward the proximal end side.

Therefore, by operating either the first dial operation portion 74 or the first slider operation portion 81, an operation of individually pulling either the first operation wire 41 (or the third operation wire 43) or the second operation wire 42 is possible.

In addition, by operating the first dial operation portion 74 and operating the first slider operation portion 81 in parallel, an operation of simultaneously pulling the first operation wire 41 (or the third operation wire 43) and the second operation wire 42 is possible.

Here, simultaneously pulling the first operation wire 41 and the second operation wire 42 means that there is a timing at which both the first operation wire 41 and the second operation wire 42 are pulled together. This does not necessarily mean that the starting timing of pulling both the first operation wire 41 and the second operation wire 42 is the same, nor does it mean that the timing of finishing pulling both the first operation wire 41 and the second operation wire 42 is the same.

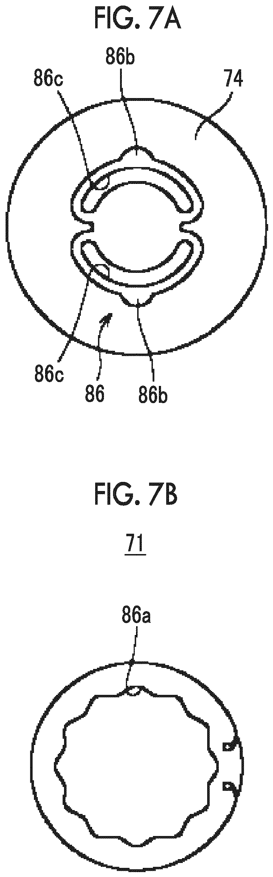

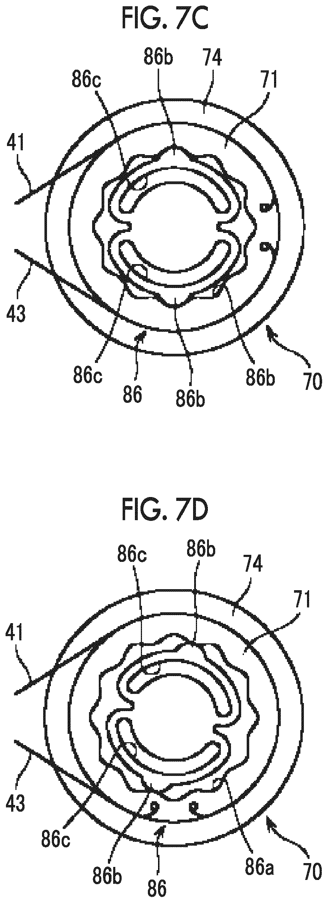

Further, the operation part 70 includes, for example, a tension release mechanism 86 (refer to FIGS. 7A to 7D) that releases a tension equal to or greater than a predetermined tension applied to the first operation wire 41.

The tension release mechanism 86 includes, for example, an engagement recessed portion 86a formed in the first rotation member 71 and an engagement protruding portion 86b formed on the first dial operation portion 74.

As shown in FIG. 7B, an opening portion penetrating the first rotation member 71 in a thickness direction is formed in a central part of the first rotation member 71. In the first rotation member 71, a peripheral edge part of the opening portion includes, for example, a plurality of recesses and protrusions in which the recesses and the protrusions are alternately disposed along a circumferential direction of the first rotation member 71, and a plurality of the engagement recessed portions 86a are formed by the recesses and the protrusions.

As shown in FIG. 7A, in the case of the present embodiment, a pair of engagement protruding portions 86b are formed on the first dial operation portion 74. Each of the pair of engagement protruding portions 86b is formed in a shape of protruding toward a radially outer side. The pair of engagement protruding portions 86b are disposed at positions that are 180 degrees rotationally symmetric to each other with respect to an axial center of the first dial operation portion 74 as a reference. In addition, a pair of slit portions 86c penetrating the first dial operation portion 74 in a thickness direction is formed in the first dial operation portion 74. The pair of slit portions 86c are disposed at positions that are 180 degrees rotationally symmetric to each other with respect to the axial center of the first dial operation portion 74 as a reference. When viewed in the axial direction of the first dial operation portion 74, each slit portion 86c forms, for example, an arc shape protruding toward the radially outer side. Each slit portion 86c is formed, whereby the first dial operation portion 74 is elastically deformable toward a radially inner side.

As shown in FIG. 7D, each engagement protruding portion 86b is engaged with one engagement recessed portion 86a of the plurality of engagement recessed portions 86a.

Next, motions will be described.

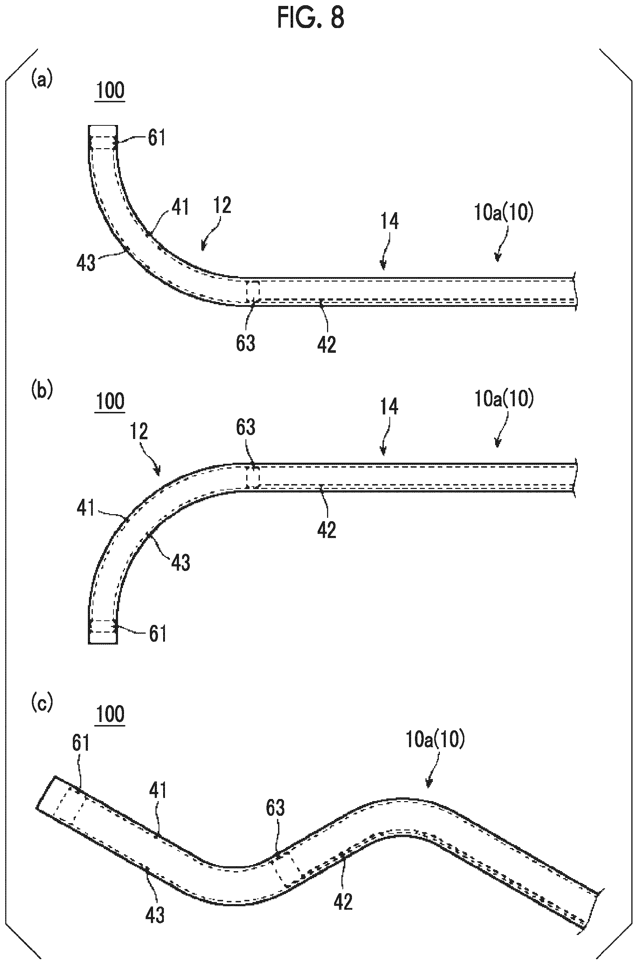

First, in a case where the operation of selectively pulling the first operation wire 41 toward the proximal end side is performed, a tension is applied to the first operation wire 41, and as shown in (a) of FIG. 8, the distal end part 10a of the medical device body 10 is bent toward a side (hereinafter, the first direction) of the first hollow tube 31 through which the first operation wire 41 is inserted, with the axial core of the medical device body 10 as a reference.

In addition, in a case where the operation of selectively pulling the third operation wire 43 toward the proximal end side is performed, a tension is applied to the third operation wire 43, and as shown in (b) of FIG. 8,, the distal end part 10a of the medical device body 10 is bent toward a side (hereinafter, the second direction) of the third hollow tube 33 through which the third operation wire 43 is inserted, with the axial core of the medical device body 10 as a reference. Here, for example, the first direction and the second direction are directions included in the same plane (hereinafter, a first virtual plane) and are directions opposite to each other.

Further, in a case where the operation of simultaneously pulling both the first operation wire 41 and the second operation wire 42 is performed, as shown in (c) of FIG. 8, the first region 12 is primarily bent in the first direction, and the second region 14 is primarily bent in the second direction. That is, in a case where a tension is applied to the second operation wire 42, the distal end part 10a of the medical device body 10 is bent toward a side (third hollow tube 33 side) of the second hollow tube 32 through which the second operation wire 42 is inserted, with the axial core of the medical device body 10 as a reference in a region on the proximal end side with respect to the first region 12. Therefore, a combined motion occurs in which a motion of primarily bending the second region 14 in the second direction by pulling the second operation wire 42 and a motion of primarily bending the first region 12 in the first direction by pulling the first operation wire 41.

The operation of pulling the second operation wire 42 may be performed alone. In this case, the second region 14 is primarily bent in the second direction.

As described above, according to the present embodiment, the operation of selectively bending the distal end part 10a of the medical device body 10 in the first direction, the operation of selectively bending the distal end part 10a of the medical device body 10 in the second direction, and the operation of primarily bending the first region 12 in the first direction and primarily bending the second region 14 in the second direction are possible. Therefore, the branching selectivity of the medical device 100 at each branching part of the body cavity (the blood vessel or the like) is improved.

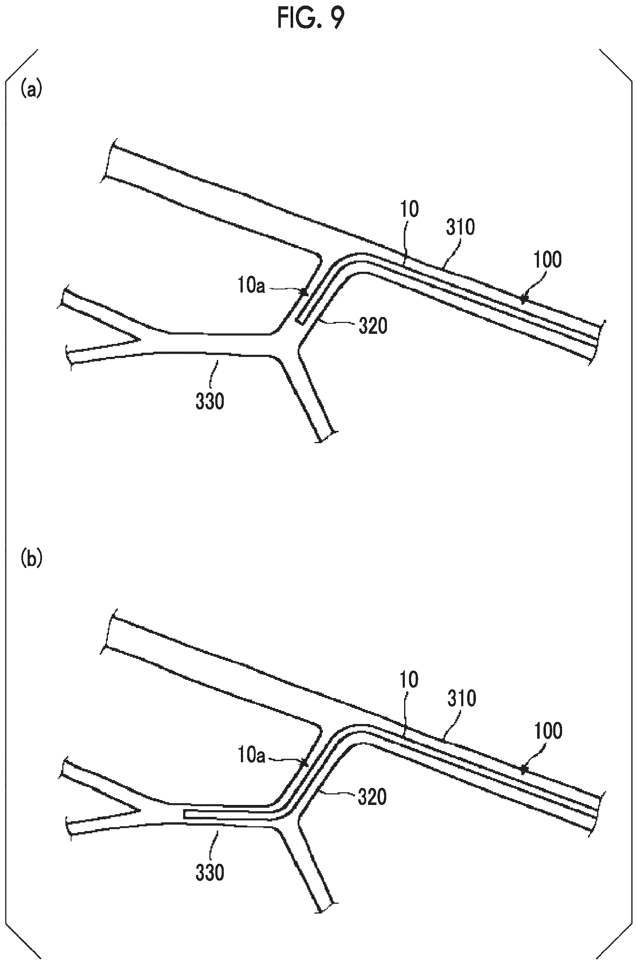

More specifically, for example, when inserting the distal end part 10a into each branching part (branching parts 310 to 330 shown in FIG. 9) of the body cavity (the blood vessel or the like), the distal end part 10a is bent in the second direction by the operation of pulling the third operation wire 43 at a timing at which the distal end of the distal end part 10a approaches the second branching part 320 that branches from the first branching part 310, as shown in (a) of FIG. 9. Consequently, the distal end part 10a can be inserted into the second branching part 320. Subsequently, at a timing at which the distal end of the distal end part 10a approaches the third branching part 330 that branches from the second branching part 320, the pulling with respect to the third operation wire 43 is released, and the distal end part 10a (primarily the second region 14) is bent in the second direction by the operation of pulling the second operation wire 42, thereby entering a backed-up state. More specifically, even when attempting to perform a torque rotation operation of rotating the medical device body 10 around the axis in a state in which the second operation wire 42 is pulled toward the proximal end side, the distal end part 10a tends to maintain a state in which the second hollow tube 32 (second operation wire 42) side is located most inwardly (in-course) of the curvature with the axial core of the medical device body 10 as a reference, so that the rotation by the rotation operation is less likely to be transmitted to the medical device body 10. In other words, the medical device body 10 is restrained from rotating around the axis, thereby entering a backed-up state.

Then, by pulling the first operation wire 41 while maintaining a state in which the second operation wire 42 is pulled from this state, the distal end part 10a is bent in a substantially S-like shape and inserted into the third branching part 330 as shown in (b) of FIG. 9.

Here, in the case of the present embodiment, as described above, the breaking strength of the second operation wire 42 is greater than the breaking strength of the first operation wire 41.

Consequently, when simultaneously pulling the first operation wire 41 and the second operation wire 42, the first region 12 can be primarily bent in the first direction while maintaining a state in which the second region 14 is primarily bent in the second direction.

Further, in the case of the present embodiment, as described above, the medical device body 10 includes the reinforcing layer 51. The bending stiffness of the reinforcing layer 51 on the proximal end side with respect to the second marker 63 is greater than the bending stiffness of the reinforcing layer 51 on the distal end side with respect to the second marker 63. The bending stiffness of the medical device body 10 on the proximal end side with respect to the second marker 63 is greater than the bending stiffness of the medical device body 10 on the distal end side with respect to the second marker 63.

As a result, more reliably, the first region 12 can be primarily bent in the first direction while maintaining a state in which the second region 14 is primarily bent in the second direction when simultaneously pulling the first operation wire 41 and the second operation wire 42.

Here, in the case of the present embodiment, the maximum value of the tension applicable to the second operation wire 42 by the pulling by the operation part 70 is greater than the maximum value of the tension applicable to the first operation wire 41 by the pulling by the operation part 70.

Consequently, the backup capability of the first operation wire 41 with respect to the second operation wire 42 and the third operation wire 43 can be sufficiently ensured. Therefore, more reliably, the first region 12 can be primarily bent in the first direction while maintaining a state in which the second region 14 is primarily bent in the second direction when simultaneously pulling the first operation wire 41 and the second operation wire 42.

More specifically, in the case of the present embodiment, when attempting to pull the first operation wire 41 with a tension equal to or higher than a certain level, the engagement between the engagement recessed portion 86a and the engagement protruding portion 86b is temporarily released, and the first dial operation portion 74 is configured to rotate without engagement with respect to the first rotation member 71. That is, the tension when the first dial operation portion 74 rotates without engagement with respect to the first rotation member 71 is the maximum value of the tension applicable to the first operation wire 41.

In more detail, as described above, since the distal end of the first operation wire 41 is fixed to the first marker 61, when the force exerted by the operation part 70 to pull the first operation wire 41 toward the proximal end side increases, the force to pull back the first operation wire 41 toward the distal end side also increases. Then, when the force to pull back the first operation wire 41 toward the distal end side is equal to or higher than a certain level, the first rotation member 71 is elastically deformed toward the radially inner side, and the engagement between the engagement recessed portion 86a and the engagement protruding portion 86b is temporarily released. Consequently, the first dial operation portion 74 rotates without engagement with respect to the first rotation member 71 (the rotation of the first dial operation portion 74 is not transmitted to the first rotation member 71).

When the force to pull the first operation wire 41 toward the proximal end side and the force to pull back the first operation wire 41 toward the distal end side reach an equilibrium state, the engagement recessed portion 86a and the engagement protruding portion 86b are engaged with each other, thereby consequently maintaining the engagement state between the first rotation member 71 and the first dial operation portion 74.

Here, in the case of the present embodiment, the medical device 100 is configured such that the maximum value of the tension applicable to the first operation wire 41 (the tension when the first dial operation portion 74 rotates without engagement with respect to the first rotation member 71) is smaller than the tension applied to the second operation wire 42 when the first slider operation portion 81 is slid toward the proximal end side to the maximum. Therefore, it is possible to restrain the tension applied to the first operation wire 41 by the pulling by the operation part 70 from exceeding the maximum value of the tension applicable to the second operation wire 42 by the pulling by the operation part 70.

In the case of the present embodiment, the maximum value of the tension applicable to the first operation wire 41 by the pulling by the operation part 70 can be set as appropriate, for example, based on the depth dimension (radial dimension) of the engagement recessed portion 86a, the depth dimension or the length dimension (circumferential dimension) of the slit portion 86c, the elastic force (spring force) of the first rotation member 71, and the like.

In addition, the maximum value of the tension applicable to the second operation wire 42 by the pulling by the operation part 70 can be set as appropriate, for example, based on the maximum stroke of the first slider operation portion 81 (the axial dimension of the slide hole).

Similarly, the maximum value of the tension applicable to the second operation wire 42 by the pulling by the operation part 70 is greater than the maximum value of the tension applicable to the third operation wire 43 by the pulling by the operation part 70.

More specifically, the medical device 100 is configured such that the maximum value of the tension applicable to the third operation wire 43 (the tension when the first dial operation portion 74 rotates without engagement with respect to the first rotation member 71) is smaller than the tension applied to the second operation wire 42 when the first slider operation portion 81 is slid toward the proximal end side to the maximum.

### [Second Embodiment]

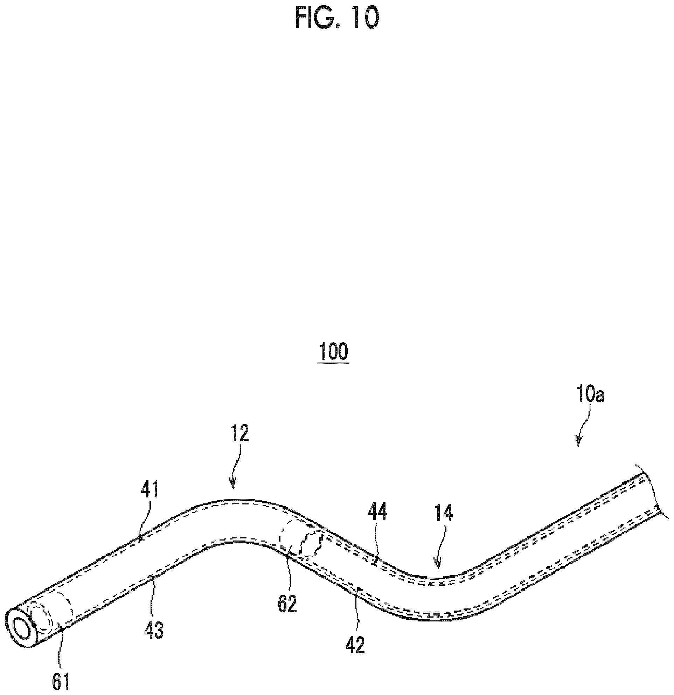

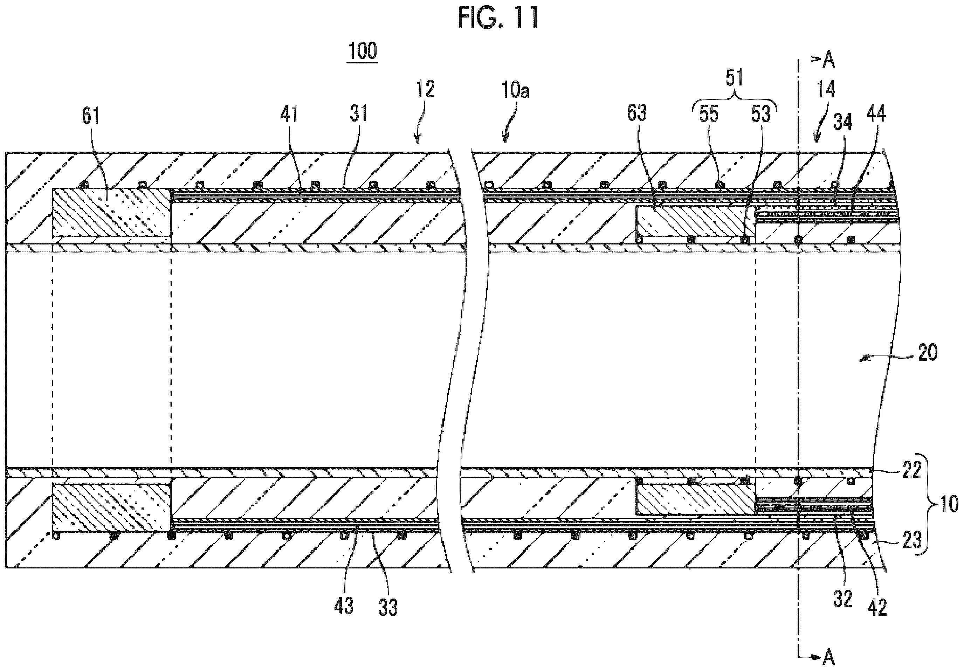

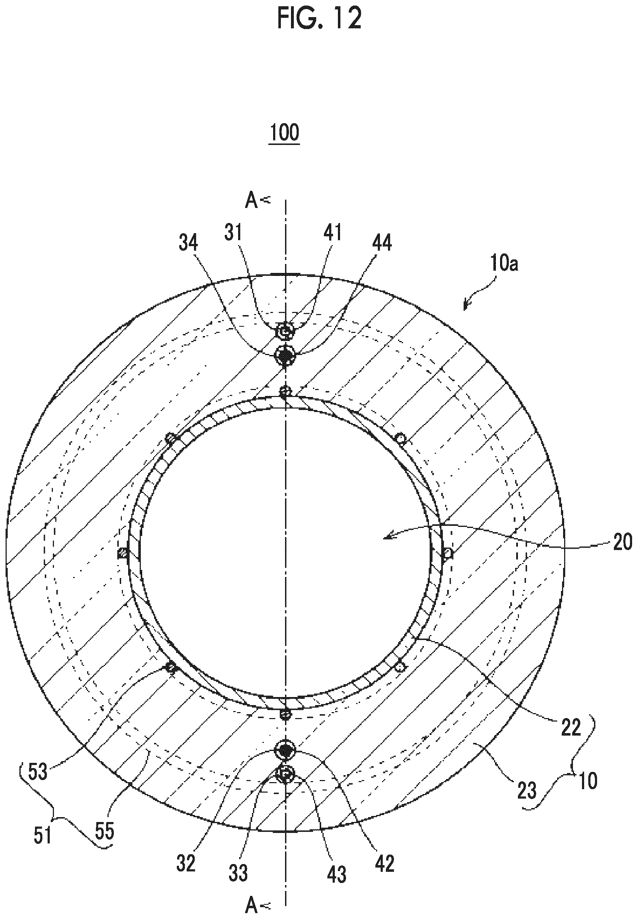

Next, a second embodiment will be described using FIGS. 10 to 13. FIG. 11 is a sectional view taken along line A-A of FIG. 12.

The medical device 100 according to the present embodiment is different from the medical device 100 according to the above-described first embodiment in the points to be described below and is configured similarly to the medical device 100 according to the above-described first embodiment in other points.

In the case of the present embodiment, the medical device 100 further includes a fourth operation wire 44 that extends along the longitudinal direction of the medical device body 10.

As shown in FIGS. 11 and 12, the fourth operation wire 44 is disposed at a position near the first operation wire 41 in the transverse section of the medical device body 10, and a distal end of the fourth operation wire 44 is fixed to the distal end part 10a of the medical device body 10 at a position on the proximal end side with respect to the distal end of the third operation wire 43. A breaking strength of the fourth operation wire 44 is greater than the breaking strength of the third operation wire 43.

With such a configuration, by simultaneously pulling the third operation wire 43 and the fourth operation wire 44, as shown in FIG. 10, the first region 12 can be primarily bent in the second direction, and the second region 14 can be primarily bent in the first direction. That is, since an operation similar to the bending operation performed by the operation of simultaneously pulling the first operation wire 41 and the second operation wire 42 (here, motions that are 180 degrees rotationally symmetric to each other with respect to the axial core of the medical device body 10 as a reference) can be achieved even with an operation of simultaneously pulling the third operation wire 43 and the fourth operation wire 44, a need to perform the torque rotation operation of rotating the medical device body 10 around the axis prior to the bending operation can be eliminated, or the rotation amount in the torque rotation operation can be reduced. Therefore, the operability of the medical device 100 can be improved.

In FIG. 11, the first operation wire 41 to the fourth operation wire 44 are shown by dashed lines.

In the case of the present embodiment, as shown in FIGS. 11 and 12, the third operation wire 43 and the fourth operation wire 44 are disposed at opposite extreme positions in the transverse section of the medical device body 10.

Here, the opposite extreme positions mean that the third operation wire 43 and the fourth operation wire 44 are spaced apart from each other by 160 degrees or more in the circumferential direction of the medical device body 10, and preferably, the third operation wire 43 and the fourth operation wire 44 are spaced apart from each other by 170 degrees or more in the circumferential direction of the medical device body 10.

More specifically, in the case of the present embodiment, for example, the fourth operation wire 44 and the first operation wire 41 are disposed at substantially equivalent positions in the circumferential direction of the medical device body 10 with the axial center of the medical device body 10 as a reference. Therefore, the fourth operation wire 44 is disposed at a position that is 180 degrees rotationally symmetric to the second operation wire 42 and the third operation wire 43 with respect to the axial center of the medical device body 10 as a reference.

In the case of the present embodiment, a direction in which the distal end part 10a of the medical device body 10 is bent by pulling the fourth operation wire 44 and a direction in which the distal end part 10a of the medical device body 10 is bent by pulling the third operation wire 43 (or the second operation wire 42) are directions opposite to each other.

As shown in FIGS. 10 and 11, in the case of the present embodiment, the medical device 100 includes a fourth hollow tube 34 that is embedded in the outer layer 23, and the fourth operation wire 44 is inserted through the fourth hollow tube 34.

The fourth hollow tube 34 is also a sub-lumen tube, and the inner lumen of the sub-lumen tube is a sub-lumen, similar to the first hollow tube 31 to the third hollow tube 33. That is, the fourth operation wire 44 is inserted through the sub-lumen.

An inner diameter of the fourth hollow tube 34 is smaller than the inner diameter of the lumen 20.

The fourth operation wire 44 is made of a thin wire such as a metal or a resin, similar to the first operation wire 41 to the third operation wire 43.

A wire diameter of the fourth operation wire 44 is set to be, for example, substantially equivalent to the wire diameter of the second operation wire 42. Therefore, the wire diameter of the fourth operation wire 44 is larger than the wire diameter of the first operation wire 41 and the wire diameter of the third operation wire 43.

The wire diameter of the fourth operation wire 44 is not particularly limited, but is, for example, preferably 30 µm or more and 180 µm or less and more preferably 60 µm or more and 150 µm or less.

The distal end of the fourth operation wire 44 and the distal end of the fourth hollow tube 34 are fixed to the proximal end of the second marker 63. Therefore, the distal end of the second operation wire 42 and the distal end of the fourth operation wire 44 are fixed at equivalent positions in the longitudinal direction of the medical device body 10. The distal end of the first operation wire 41 and the distal end of the third operation wire 43 are fixed at positions on the distal end side with respect to the positions where the distal end of the second operation wire 42 and the distal end of the fourth operation wire 44 are fixed.

In the case of the present embodiment, in addition to the above-described operations, an operation of individually pulling the fourth operation wire 44 toward the proximal end side and an operation of simultaneously pulling the third operation wire 43 and the fourth operation wire 44 toward the proximal end side can be performed with respect to the operation part 70.

Here, simultaneously pulling the third operation wire 43 and the fourth operation wire 44 means that there is a timing at which both the third operation wire 43 and the fourth operation wire 44 are pulled together. This does not necessarily mean that the starting timing of pulling both the third operation wire 43 and the fourth operation wire 44 is the same, nor does it mean that the timing of finishing pulling both the third operation wire 43 and the fourth operation wire 44 is the same.

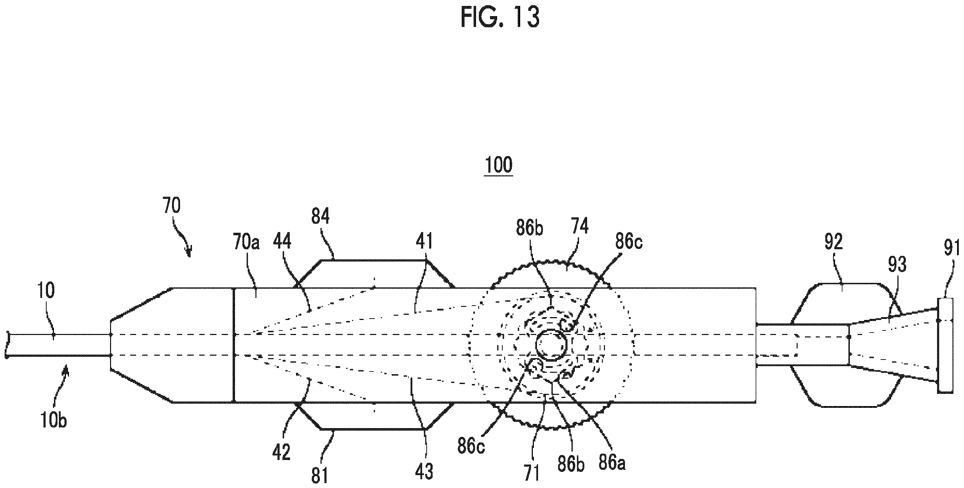

More specifically, as shown in FIG. 13, the operation part 70 further includes a second slider operation portion 84 that is fixed to the body case 70a to be slidable in the axial direction.

The second slider operation portion 84 is configured similarly to the first slider operation portion 81, for example. Therefore, a slide hole (not shown) for guiding the second slider operation portion 84 along the axial direction is formed in the body case 70a. The first slider operation portion 81 and the second slider operation portion 84 are disposed at positions that are 180 degrees rotationally symmetric to each other with respect to the axial core of the medical device body 10 as a reference.

As shown in FIG. 13, at least a portion of the second slider operation portion 84 is exposed to the outside of the body case 70a, and the operator who operates the medical device 100 can perform an operation of sliding the second slider operation portion 84 from the outside of the body case 70a.

A proximal end of the fourth operation wire 44 is fixed to, for example, the second slider operation portion 84.

In a case where the second slider operation portion 84 is slid toward the proximal end side relative to the body case 70a in the axial direction through the operation with respect to the second slider operation portion 84, the fourth operation wire 44 is pulled toward the proximal end side.

Therefore, by operating the first dial operation portion 74 and operating the second slider operation portion 84 in parallel, an operation of simultaneously pulling the third operation wire 43 and the fourth operation wire 44 is possible.

Here, in the case of the present embodiment, the maximum value of the tension applicable to the fourth operation wire 44 by the pulling by the operation part 70 is greater than the maximum value of the tension applicable to the third operation wire 43 (first operation wire 41) by the pulling by the operation part 70.

Consequently, by simultaneously pulling the third operation wire 43 and the fourth operation wire 44, more reliably, the first region 12 can be primarily bent in the second direction, and the second region 14 can be primarily bent in the first direction.

More specifically, a configuration is employed in which a tension required to rotate the first dial operation portion 74 without engagement is smaller than the tension applied to the fourth operation wire 44 when the second slider operation portion 84 is slid toward the proximal end side to the maximum. As a result, it is possible to restrain the tension applied to the third operation wire 43 by the pulling by the operation part 70 from exceeding the maximum value of the tension applicable to the fourth operation wire 44 by the pulling by the operation part 70.

### [Third Embodiment]

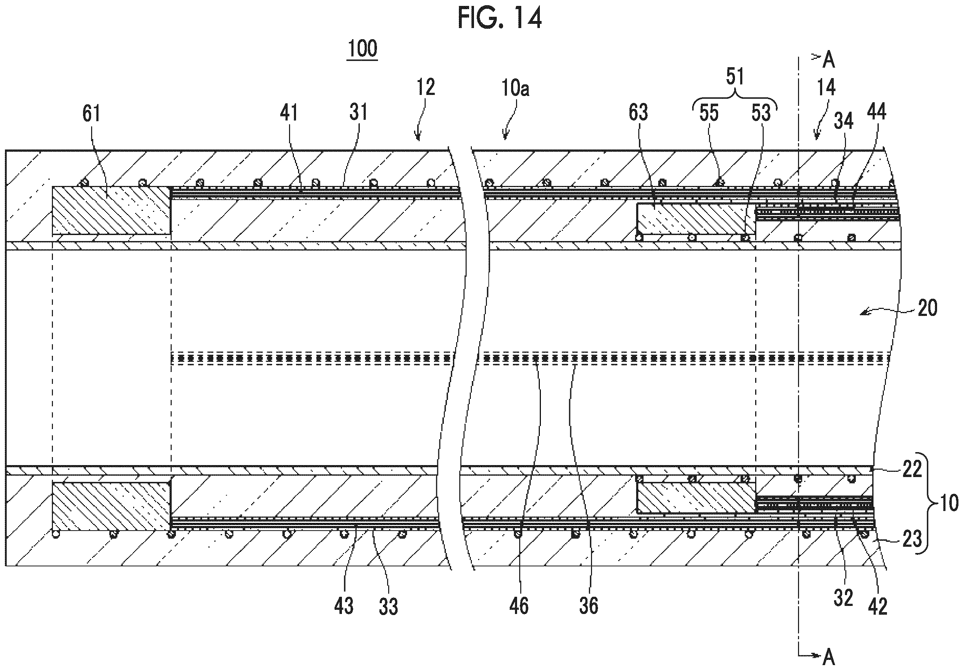

Next, a third embodiment will be described using FIGS. 14 to 18.

The medical device 100 according to the present embodiment is different from the medical device 100 according to the above-described first and second embodiments in the points to be described below and is configured similarly to the medical device 100 according to the above-described first and second embodiments in other points.

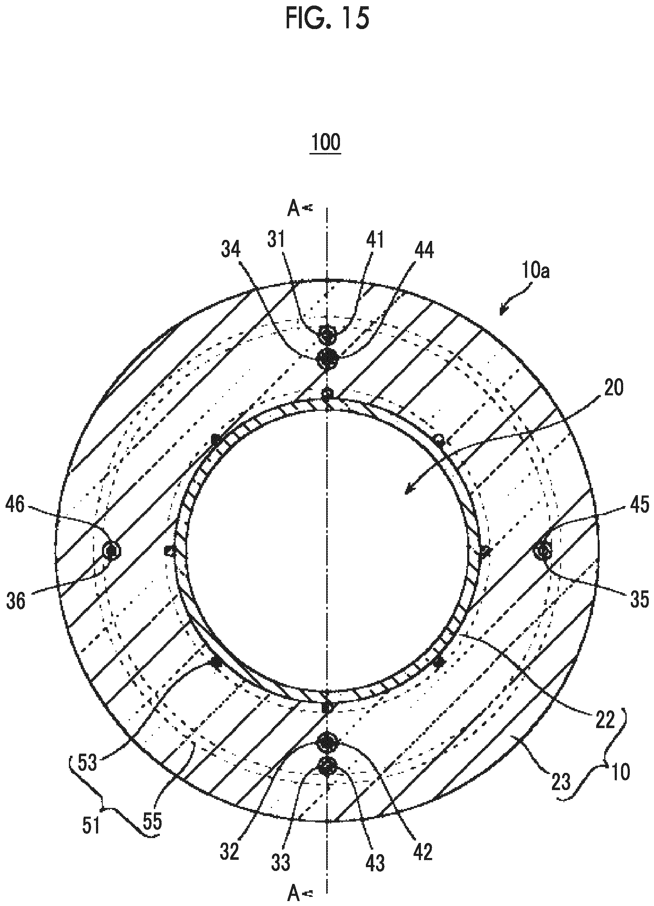

As shown in FIGS. 14 and 15, in the case of the present embodiment, the medical device 100 further includes a fifth operation wire 45 that extends along the longitudinal direction of the medical device body 10.

The fifth operation wire 45 is disposed in a region between the disposition region of the first operation wire 41 and the fourth operation wire 44 and the disposition region of the second operation wire 42 and the third operation wire 43 in the circumferential direction of the transverse section of the medical device body 10.

A distal end of the fifth operation wire 45 is fixed to the distal end part 10a of the medical device body 10 at a position on the distal end side with respect to the distal end of the second operation wire 42 and the distal end of the fourth operation wire 44.

With such a configuration, by pulling the fifth operation wire 45 toward the proximal end side, the distal end part 10a of the medical device body 10 can be bent in a direction different from the first direction and the second direction, so that the branching selectivity of the medical device 100 at each branching part of the body cavity (the blood vessel or the like) can be further improved.

Further, when inserting the distal end part 10a into each branching part of the body cavity (the blood vessel or the like), a need to perform the torque rotation operation of rotating the medical device body 10 around the axis prior to the bending operation can be eliminated, or the rotation amount in the torque rotation operation can be reduced. Therefore, the operability of the medical device 100 can be improved.

Here, in the case of the present embodiment, the fifth operation wire 45 is preferably disposed at a position that is at least approximately 45 degrees different from the disposition region of the first operation wire 41 and the fourth operation wire 44 (or the disposition region of the second operation wire 42 and the third operation wire 43) and more preferably disposed at a position that is at approximately 90 degrees different, in the circumferential direction.

The distal end of the fifth operation wire 45 and the distal end of the first operation wire 41 (and the third operation wire 43) are fixed at equivalent positions in the longitudinal direction of the medical device body 10.

As shown in FIG. 15, as an example, the fifth operation wire 45, and the first operation wire 41 and the third operation wire 43 are disposed at positions different from each other by approximately 90 degrees in the circumferential direction of the medical device body 10. Similarly, as an example, the fifth operation wire 45, and the third operation wire 43 and the fourth operation wire 44 are disposed at positions different from each other by approximately 90 degrees in the circumferential direction of the medical device body 10.

As shown in FIG. 15, in the case of the present embodiment, the medical device 100 further includes, for example, a sixth operation wire 46 that extends along the longitudinal direction of the medical device body 10.

The distal end of the fifth operation wire 45 and a distal end of the sixth operation wire 46 are fixed at equivalent positions in the longitudinal direction of the medical device body 10. Therefore, the distal end of the sixth operation wire 46 and the distal end of the first operation wire 41 (and the third operation wire 43) are fixed at equivalent positions in the longitudinal direction of the medical device body 10.

The sixth operation wire 46 and the fifth operation wire 45 are disposed at positions that are 180 degrees rotationally symmetric to each other with respect to the axial center of the medical device body 10 as a reference. Therefore, the sixth operation wire 46 is disposed in a region between the disposition region of the first operation wire 41 and the fourth operation wire 44 and the disposition region of the second operation wire 42 and the third operation wire 43 in the circumferential direction of the transverse section of the medical device body 10. More specifically, the sixth operation wire 46, and the first operation wire 41 and the third operation wire 43 are spaced apart from each other by approximately 90 degrees in the circumferential direction of the medical device body 10, and the sixth operation wire 46, and the third operation wire 43 and the fourth operation wire 44 are spaced apart from each other by approximately 90 degrees in the circumferential direction of the medical device body 10.

In the case of the present embodiment, a direction in which the distal end part 10a of the medical device body 10 is bent by pulling the fifth operation wire 45 (hereinafter, a third direction) and a direction in which the distal end part 10a of the medical device body 10 is bent by pulling the sixth operation wire 46 (hereinafter, a fourth direction) are directions opposite to each other.

As shown in FIG. 15, in the case of the present embodiment, the medical device 100 includes a fifth hollow tube 35 and a sixth hollow tube 36 that are embedded in the outer layer 23. The fifth operation wire 45 is inserted through the fifth hollow tube 35, and the sixth operation wire 46 is inserted through the sixth hollow tube 36.

The fifth hollow tube 35 and the sixth hollow tube 36 are sub-lumen tubes, and the inner lumens of the sub-lumen tubes are sub-lumens, similar to the first hollow tube 31 to the third hollow tube 33.

An inner diameter of the fifth hollow tube 35 and an inner diameter of the sixth hollow tube 36 are smaller than the inner diameter of the lumen 20.

The fifth operation wire 45 and the sixth operation wire 46 are made of thin wires such as metals or resins, similar to the first operation wire 41 to the third operation wire 43.

The breaking strengths of the second operation wire 42 and the fourth operation wire 44 are larger than the breaking strengths of the fifth operation wire 45 and the sixth operation wire 46. More specifically, in the case of the present embodiment, the first operation wire 41 to the sixth operation wire 46 are made of the same type of material. However, the first operation wire 41 to the sixth operation wire 46 are made of, for example, different types of materials from each other.

Meanwhile, by setting the wire diameters of the second operation wire 42 and the fourth operation wire 44 to dimensions larger than the wire diameters of the fifth operation wire 45 and the sixth operation wire 46, the breaking strengths of the second operation wire 42 and the fourth operation wire 44 can be made greater than the breaking strengths of the fifth operation wire 45 and the sixth operation wire 46. However, for example, by using a material having a greater breaking strength than breaking strengths of materials constituting the fifth operation wire 45 and the sixth operation wire 46 as a material constituting the fourth operation wire 44, the breaking strength of the fourth operation wire 44 can also be made greater than the breaking strengths of the fifth operation wire 45 and the sixth operation wire 46.

The wire diameter of the fifth operation wire 45 and the wire diameter of the sixth operation wire 46 are not particularly limited, but are preferably 10 µm or more and 60 µm or less and more preferably 20 µm or more and 50 µm or less.

As shown in FIG. 14, the distal end of the fifth hollow tube 35 and the distal end of the fifth operation wire 45 are fixed to the proximal end of the first marker 61. Similarly, the distal end of the sixth hollow tube 36 and the distal end of the sixth operation wire 46 are fixed to the proximal end of the first marker 61.

As described above, in the case of the present embodiment, the number of operation wires whose distal ends are fixed at positions on the distal end side with respect to the positions where the distal end of the second operation wire 42 and the distal end of the fourth operation wire 44 are fixed is equal to or greater than the number of operation wires whose distal ends are fixed at equivalent positions to the second operation wire 42 and the fourth operation wire 44.

Here, the equivalent position means that the spaced distance between the distal ends of the respective operation wires in the axial direction of the medical device body 10 is smaller than the outer diameter of the medical device body 10. In addition, as in the present embodiment, in a case where the distal ends of the respective operation wires are fixed to a common marker (the first marker 61 or the second marker 63), the axial dimension of the marker may be larger than the outer diameter of the medical device body 10, but is preferably smaller than the outer diameter of the medical device body 10.

In the case of the present embodiment, in addition to the above-described operations, an operation of selectively pulling either the fifth operation wire 45 or the sixth operation wire 46 toward the proximal end side, an operation of individually and simultaneously pulling the fifth operation wire 45 and the second operation wire 42 (or the fourth operation wire 44), and an operation of individually and simultaneously pulling the sixth operation wire 46 and the second operation wire 42 (or the fourth operation wire 44) are possible with respect to the operation part 70.

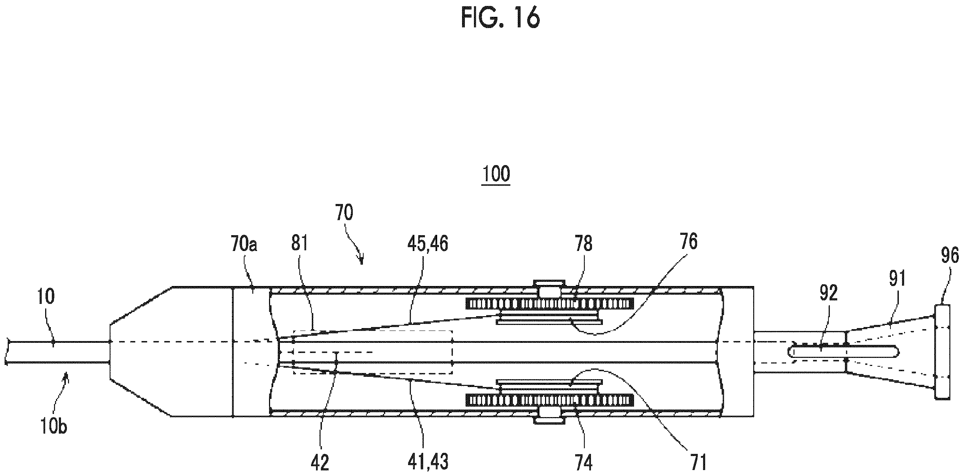

More specifically, as shown in FIG. 16, the operation part 70 includes a second rotation member 76 that is pivotally supported to be rotatable with respect to the body case 70a, and a second dial operation portion 78 that is fixed to the second rotation member 76.

The second rotation member 76 is configured similarly to the first rotation member 71, for example. The second dial operation portion 78 is configured similarly to the first dial operation portion 74, for example.

In the example shown in FIG. 16, the first dial operation portion 74 and the second dial operation portion 78 are disposed to overlap each other in a direction orthogonal to their plate surfaces, and the rotary shaft of the first dial operation portion 74 and a rotary shaft of the second dial operation portion 78 are coaxially disposed.

However, the rotary shaft of the first dial operation portion 74 and the rotary shaft of the second dial operation portion 78 need not be coaxially disposed. For example, the first dial operation portion 74 and the second dial operation portion 78 may be disposed at positions different from each other in the axial direction of the medical device body 10.

In the case of the present embodiment, a proximal end part of the fifth operation wire 45 is, for example, wound around the second rotation member 76 by one and a half turns, and a proximal end of the fifth operation wire 45 is fixed to the second rotation member 76.

In addition, a proximal end part of the sixth operation wire 46 is, for example, wound around the second rotation member 76 by one and a half turns, and a proximal end of the sixth operation wire 46 is fixed to the second rotation member 76.

A winding direction of the fifth operation wire 45 and a winding direction of the sixth operation wire 46 with respect to the second rotation member 76 are directions opposite to each other.

In a case where the second rotation member 76 that is engaged with the second dial operation portion 78 is rotated in one direction by a rotation operation with respect to the second dial operation portion 78, the fifth operation wire 45 out of the fifth operation wire 45 and the sixth operation wire 46 is selectively pulled toward the proximal end side. Then, the distal end part 10a of the medical device body 10 is bent toward a side of the fifth hollow tube 35 through which the fifth operation wire 45 is inserted, with the axial core of the medical device body 10 as a reference. Here, as described above, a plane including the direction (first direction) in which the distal end part 10a is bent by pulling the first operation wire 41 and the direction (second direction) in which the distal end part 10a is bent by pulling the second operation wire 42 is referred to as the first virtual plane. The direction (referred to as the third direction) in which the distal end part 10a is bent by pulling the fifth operation wire 45 is, for example, a direction included in a second virtual plane orthogonal to the first virtual plane.

In addition, in a case where the second rotation member 76 is rotated in the opposite direction to the above-described one direction by the rotation operation with respect to the second dial operation portion 78, the sixth operation wire 46 out of the fifth operation wire 45 and the sixth operation wire 46 is selectively pulled toward the proximal end side. Then, the distal end part 10a of the medical device body 10 is bent toward a side of the sixth hollow tube 36 through which the sixth operation wire 46 is inserted, with the axial core of the medical device body 10 as a reference. That is, the distal end part 10a of the medical device body 10 is bent in the opposite direction (fourth direction) to the third direction with the axial core of the medical device body 10 as a reference. The fourth direction is also a direction included in the second virtual plane.

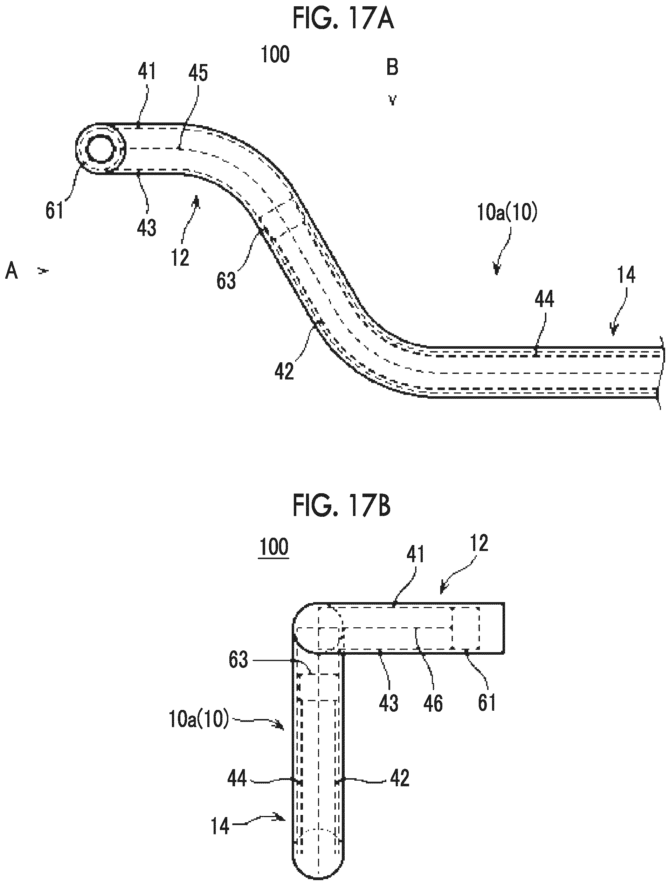

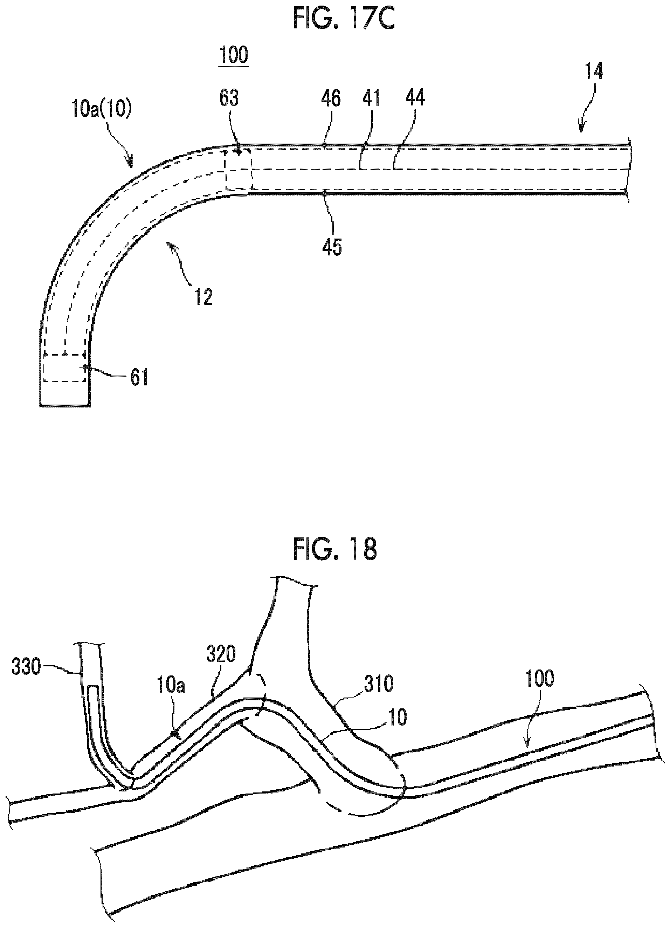

Further, in a case where an operation of pulling both the fifth operation wire 45 and the second operation wire 42 (or the fourth operation wire 44) is performed, as shown in FIGS. 17A to 17C, the first region 12 is primarily bent in the third direction, and the second region 14 is primarily bent in the second direction (or the first direction). That is, in the first and second embodiments, the bending of the distal end part 10a is two-dimensional bending in the above-described first virtual plane, but in the case of the present embodiment, it is possible to achieve a so-called three-dimensional (stereoscopic) bending of the distal end part 10a by combining the bending in the first virtual plane and the bending in the second virtual plane.

In addition, in a case where an operation of pulling both the sixth operation wire 46 and the second operation wire 42 (or the fourth operation wire 44) is performed, the first region 12 is primarily bent in the fourth direction, and the second region 14 is primarily bent in the second direction (or the first direction). That is, in this case as well, the distal end part 10a can be three-dimensionally (stereoscopically) bent.

As described above, according to the present embodiment, the distal end part 10a of the medical device body 10 can be three-dimensionally (stereoscopically) bent by the operation of simultaneously pulling the fifth operation wire 45 and the second operation wire 42 (or the fourth operation wire 44) or the operation of simultaneously pulling the sixth operation wire 46 and the second operation wire 42 (or the fourth operation wire 44). Therefore, for example, as shown in FIG. 18, the distal end part 10a of the medical device body 10 can also be inserted along each branching part (the first branching part 310 to the third branching part 330) of the body cavity (the blood vessel or the like) that branches three-dimensionally (stereoscopically) for each branching part.

Here, in the case of the present embodiment, the maximum value of the tension applicable to the second operation wire 42 (or the fourth operation wire 44) by the pulling by the operation part 70 is greater than the maximum value of the tension applicable to the fifth operation wire 45 by the pulling by the operation part 70.

This allows for sufficient backup, and by simultaneously pulling the fifth operation wire 45 and the second operation wire 42 (or the fourth operation wire 44), more reliably, the first region 12 can be primarily bent in the third direction, and the second region 14 can be primarily bent in the second direction (or the first direction).

Similarly, in the case of the present embodiment, the maximum value of the tension applicable to the second operation wire 42 (or the fourth operation wire 44) by the pulling by the operation part 70 is greater than the maximum value of the tension applicable to the sixth operation wire 46 by the pulling by the operation part 70.

This allows for sufficient backup, and by simultaneously pulling the sixth operation wire 46 and the second operation wire 42 (or the fourth operation wire 44), more reliably, the first region 12 can be primarily bent in the fourth direction, and the second region 14 can be primarily bent in the first direction (or the second direction).

More specifically, the operation part 70 includes, for example, a tension release mechanism (not shown) that releases a tension equal to or greater than a predetermined tension applied to the fifth operation wire 45 (or the sixth operation wire 46).

The tension release mechanism is configured similarly to the tension release mechanism 86, for example.

Therefore, the medical device 100 is configured such that the maximum value of the tension applicable to the fifth operation wire 45 (or the sixth operation wire 46) (the tension when the second dial operation portion 78 rotates without engagement with respect to the second rotation member 76) is smaller than the tension applied to the second operation wire 42 when the first slider operation portion 81 is slid toward the proximal end side to the maximum.

Each embodiment has been described with reference to the drawings; however, these are illustrative examples of the present invention, and various configurations other than those described above can also be employed.

In addition, the respective embodiments described above can be appropriately combined within a range that does not depart from the gist of the present invention.

The present embodiment encompasses the following technical concepts.
(1) A medical device including:
   an elongated medical device body;
   a first operation wire and a second operation wire, each of which extends along a longitudinal direction of the medical device body; and
   an operation part that is configured to individually and simultaneously pull the first operation wire and the second operation wire,
   in which the first operation wire and the second operation wire are disposed at opposite extreme positions in a transverse section of the medical device body,
   a distal end of the first operation wire is fixed to a distal end part of the medical device body, and
   a distal end of the second operation wire is fixed to the distal end part of the medical device body at a position on a proximal end side with respect to the distal end of the first operation wire.
(2) The medical device according to (1),
   in which a breaking strength of the second operation wire is greater than a breaking strength of the first operation wire.
(3) The medical device according to (1) or (2),
   in which a maximum value of a tension applicable to the second operation wire by the pulling by the operation part is greater than a maximum value of a tension applicable to the first operation wire by the pulling by the operation part.
(4) The medical device according to any one of (1) to (3), further comprising:
   a third operation wire that extends along the longitudinal direction of the medical device body,
   in which the third operation wire is disposed at a position near the second operation wire in the transverse section of the medical device body, and
   a distal end of the third operation wire is fixed to the distal end part of the medical device body at a position on a distal end side with respect to the distal end of the second operation wire.
(5) The medical device according to (4), further comprising:
   a fourth operation wire that extends along the longitudinal direction of the medical device body,
   in which the fourth operation wire is disposed at a position near the first operation wire in the transverse section of the medical device body,
   a distal end of the fourth operation wire is fixed to the distal end part of the medical device body at a position on the proximal end side with respect to the distal end of the third operation wire, and
   a breaking strength of the fourth operation wire is greater than a breaking strength of the third operation wire.
(6) The medical device according to (5), further comprising:
   a fifth operation wire that extends along the longitudinal direction of the medical device body,
   in which the fifth operation wire is disposed in a region between a disposition region of the first operation wire and the fourth operation wire and a disposition region of the second operation wire and the third operation wire in a circumferential direction of the transverse section of the medical device body, and
   a distal end of the fifth operation wire is fixed to the distal end part of the medical device body at a position on the distal end side with respect to the distal end of the second operation wire and the distal end of the fourth operation wire.
(7) The medical device according to (6),
   in which the distal end of the second operation wire and the distal end of the fourth operation wire are fixed at equivalent positions in the longitudinal direction of the medical device body, and
   the number of operation wires whose distal ends are fixed at positions on the distal end side with respect to the positions where the distal end of the second operation wire and the distal end of the fourth operation wire are fixed is equal to or greater than the number of operation wires whose distal ends are fixed at the equivalent positions to the second operation wire and the fourth operation wire.
(8) The medical device according to any one of (4) to (7), further comprising:
   a first marker that is fixed to the distal end part of the medical device body; and
   a second marker that is fixed to the distal end part of the medical device body at a position on the proximal end side with respect to the first marker,
   in which the distal end of the first operation wire and the distal end of the third operation wire are fixed to the first marker, and
   the distal end of the second operation wire is fixed to the second marker.
(9) The medical device according to (8),
   in which the medical device body includes a reinforcing layer,
   a bending stiffness of the reinforcing layer on the proximal end side with respect to the second marker is greater than a bending stiffness of the reinforcing layer on the distal end side with respect to the second marker, and
   a bending stiffness of the medical device body on the proximal end side with respect to the second marker is greater than a bending stiffness of the medical device body on the distal end side with respect to the second marker.
(10) The medical device according to any one of (1) to (9),
   in which the medical device is a catheter, and
   the medical device body has a lumen that is open at a distal end of the medical device body.

### Reference Signs List

10: medical device body
10a: distal end part
10b: proximal end part
12: first region
14: second region
20: lumen
22: inner layer
23: outer layer
31: first hollow tube
32: second hollow tube
33: third hollow tube
34: fourth hollow tube
35: fifth hollow tube
36: sixth hollow tube
41: first operation wire
42: second operation wire
43: third operation wire
44: fourth operation wire
45: fifth operation wire
46: sixth operation wire
51: reinforcing layer
53: blade layer
55: winding wire
61: first marker
63: second marker
70: operation part
70a: body case
71: first rotation member
74: first dial operation portion
76: second rotation member
78: second dial operation portion
81: first slider operation portion
84: second slider operation portion
86: tension release mechanism
86a: engagement recessed portion
86b: engagement protruding portion
86c: slit portion
91: hub
92: blade portion
93: coupling portion
96: gate portion
100: medical device
310: first branching part
320: second branching part
330: third branching part

## Claims

1. A medical device comprising:
an elongated medical device body;
a first operation wire and a second operation wire, each of which extends along a longitudinal direction of the medical device body; and
an operation part that is configured to individually and simultaneously pull the first operation wire and the second operation wire,
wherein the first operation wire and the second operation wire are disposed at opposite extreme positions in a transverse section of the medical device body,
a distal end of the first operation wire is fixed to a distal end part of the medical device body, and
a distal end of the second operation wire is fixed to the distal end part of the medical device body at a position on a proximal end side with respect to the distal end of the first operation wire.

2. The medical device according to Claim 1,
wherein a breaking strength of the second operation wire is greater than a breaking strength of the first operation wire.

3. The medical device according to Claim 1,
wherein a maximum value of a tension applicable to the second operation wire by the pulling by the operation part is greater than a maximum value of a tension applicable to the first operation wire by the pulling by the operation part.

4. The medical device according to Claim 1, further comprising:
a third operation wire that extends along the longitudinal direction of the medical device body,
wherein the third operation wire is disposed at a position near the second operation wire in the transverse section of the medical device body, and
a distal end of the third operation wire is fixed to the distal end part of the medical device body at a position on a distal end side with respect to the distal end of the second operation wire.

5. The medical device according to Claim 4, further comprising:
a fourth operation wire that extends along the longitudinal direction of the medical device body,
wherein the fourth operation wire is disposed at a position near the first operation wire in the transverse section of the medical device body,
a distal end of the fourth operation wire is fixed to the distal end part of the medical device body at a position on the proximal end side with respect to the distal end of the third operation wire, and
a breaking strength of the fourth operation wire is greater than a breaking strength of the third operation wire.

6. The medical device according to Claim 5, further comprising:
a fifth operation wire that extends along the longitudinal direction of the medical device body,
wherein the fifth operation wire is disposed in a region between a disposition region of the first operation wire and the fourth operation wire and a disposition region of the second operation wire and the third operation wire in a circumferential direction of the transverse section of the medical device body, and
a distal end of the fifth operation wire is fixed to the distal end part of the medical device body at a position on the distal end side with respect to the distal end of the second operation wire and the distal end of the fourth operation wire.

7. The medical device according to Claim 6,
wherein the distal end of the second operation wire and the distal end of the fourth operation wire are fixed at equivalent positions in the longitudinal direction of the medical device body, and
the number of operation wires whose distal ends are fixed at positions on the distal end side with respect to the positions where the distal end of the second operation wire and the distal end of the fourth operation wire are fixed is equal to or greater than the number of operation wires whose distal ends are fixed at the equivalent positions to the second operation wire and the fourth operation wire.

8. The medical device according to Claim 4, further comprising:
a first marker that is fixed to the distal end part of the medical device body; and
a second marker that is fixed to the distal end part of the medical device body at a position on the proximal end side with respect to the first marker,
wherein the distal end of the first operation wire and the distal end of the third operation wire are fixed to the first marker, and
the distal end of the second operation wire is fixed to the second marker.

9. The medical device according to Claim 8,
wherein the medical device body includes a reinforcing layer,
a bending stiffness of the reinforcing layer on the proximal end side with respect to the second marker is greater than a bending stiffness of the reinforcing layer on the distal end side with respect to the second marker, and
a bending stiffness of the medical device body on the proximal end side with respect to the second marker is greater than a bending stiffness of the medical device body on the distal end side with respect to the second marker.

10. The medical device according to Claim 1,
wherein the medical device is a catheter, and
the medical device body has a lumen that is open at a distal end of the medical device body.
